(19)

Europäisches
Patentamt
European
Patent Office
Office européen
des brevets

(11) **EP 3 895 563 A1**

(12) **EUROPEAN PATENT APPLICATION**
published in accordance with Art. 153(4) EPC

(43) Date of publication:
**20.10.2021 Bulletin 2021/42**

(21) Application number: **19895787.0**

(22) Date of filing: **30.04.2019**

(51) Int Cl.:
*A24F 47/00* (2020.01)     *A24B 3/14* (2006.01)

(86) International application number:
**PCT/CN2019/085099**

(87) International publication number:
**WO 2020/119011 (18.06.2020 Gazette 2020/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.12.2018 CN 201811503459**

(71) Applicant: **GUANGDONG WONDERFUL
INTERNATIONAL BIOTECHNOLOGY
CO., LTD.
Huizhou, guangdong 516517 (CN)**

(72) Inventor: **QIAN, Jianbing
Shanghai 200135 (CN)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **LOW-TEMPERATURE NON-BURNING CIGARETTE CARTRIDGE STRUCTURE**

(57)     The present invention provides a heat-not-burn
(HNB) cartridge structure, comprising a cartridge pack-
aging housing and HNB tobacco particles contained
therein. The cartridge packaging housing is composed
of a cartridge cup, a cover film and sealing films. The
cartridge cup is provided with a top opening and a bottom
end face; the cover film and the bottom end face are
provided with gas circulating holes; the top opening is
sealed by the cover film; the cover film and the bottom
end face are sealed with the sealing films at the outer
surface. An absorbent cotton is arranged at the bottom
end face in the cartridge cup. A body of the cartridge cup
has a taper. The cross section of the cartridge cup can
be round, oval, quadrilateral, polygonal or irregu-
lar-shaped. The cartridge structure of the present inven-
tion is applicable to a wide array of HNB heating smoking
sets, delivering a better compatibility between a cartridge
and a heating cavity of each smoking set as well as a
higher air tightness therebetween, thus remarkably im-
proving the heating and suction effects of the cartridge.

Fig. 3

## Description

## Technical Field

The present invention relates to the field of novel HNB tobaccos, in particular to an HNB cartridge structure.

## Background of the Invention

As science and production technologies advance, tobacco products and their usage have seen continuous innovation and refinement, making novel tobacco smoking represented by HNB a popular trend. By heating tobacco derivatives with heating components under the temperature up to 300-380°C, which is far lower than that in traditional burning way (above 800°C), HNB smoking prevents the tobacco components from being pyrolyzed under the high temperature, thus reducing the harmful substances generated. The tobacco derivatives can be cut stem, sheet or granule, which are packaged with packing materials to form tobacco products (also known as cartridge). A heating smoking set is required for HNB smoking. The smoking set is divided into needle, sheet, rod and cylinder and other types according to the heating component. Depending on different heating elements, tobacco products need to have corresponding characteristics. For example, needle-shaped heating elements are used for cartridges easy to be pierced, and cylindrical-shaped heating elements are used for cartridges with heat-transferring side walls. For this reason, there is an urgent demand of the market for a cartridge product that can both adapt to multiple heating methods and effectively guarantee the cartridge quality.

## Summary of the Invention

To meet the market demands, the present invention aims to provide a cartridge that is suitable for the non-piercing cylinder wall-type heating smoking set and various piercing-type heating smoking sets. Furthermore, the present invention is user-friendly and can ensure the long-term, stable quality of the cartridge while significantly improves the suction effect of the cartridge.

To solve the above technical problem, the present invention applies the following technical solution:
According to one aspect of the present invention, a cartridge is provided, wherein the cartridge is composed of a cartridge packaging housing and HNB tobacco particles contained therein; and the cartridge packaging housing is composed of a cartridge cup, a cover film and sealing films; The cartridge cup is provided with a top opening and a bottom end face; the cover film and the bottom end face are provided with gas circulating holes; the top opening is sealed by the cover film; the outer surface of the cover film and the bottom end face are sealed with the sealing films.

The cartridge packaging housing is composed of a cartridge cup, a cover film and sealing films; wherein the cartridge cup is provided with a top opening; the cover film is provided with gas circulating holes; the top opening is sealed by the cover film; the outer surface of the cover film at the top opening and the bottom end face are sealed by the non-porous sealing film.

The HNB tobacco particles preferably comprises microcrystalline celluloses, tobacco powder, low-temperature pyrolysis products of tobacco, a physiological taste enhancer of tobacco, an atomizing agent, a tobacco flavor and a flavor solvent. Preferably, by weight fraction, the HNB tobacco particles comprise 8-20 parts of the microcrystalline celluloses, 50-80 parts of the tobacco powder, 0.5-2 parts of the low-temperature pyrolysis products of tobacco, 1 part of the physiological taste enhancer of tobacco, 8-30 parts of the atomizing agent, 0.1-2 parts of the tobacco flavor and 1-10 parts of the flavor solvent. Preferably, the low-temperature pyrolysis products of tobacco are products of tobacco pyrolysis at 220-280°C by far-infrared heating. The physiological taste enhancer of tobacco is composed of alkaloid and water. The alkaloid is nicotine or nicotine salt, and if nicotine is selected, the physiological taste enhancer of tobacco is composed of 20-40% of the nicotine and 60-80% of the water; if nicotine salt is selected, the physiological taste enhancer of tobacco is composed of 5-20% nicotine salt and 80-95% of the water. Preferably, the atomizing agent is selected from polyol or an ester derivative of polyol. Preferably, the atomizing agent is selected from vegetable glycerin, propylene glycol or glyceryl triacetate. Preferably, the flavor solvent is selected from vegetable glycerin, propylene glycol or glyceryl triacetate.

Preferably, the top opening of the cartridge cup is provided with an outer flange radially protruding outward along the cartridge cup, and the cover film is fixed on the surface shaped by the flange.

Preferably, the top opening of the cartridge cup is hot-sealed by the cover film for sealing and fixing. More preferably, the bottom end face of the cartridge cup is provided with a breathable fibrous layer, which is preferably made of absorbent cotton. More preferably, the cover film and the sealing film at the top opening form an integrated composite film that is heat-sealed at the top opening of the cartridge cup.

The porous cover film preferably uses thermoplastic multi-layer composites composed of PE (polyethylene) and an aluminum layer.

The non-porous sealing film preferably uses adhesive multi-layer composites composed of a glue layer and a sealing layer. The cartridge is preferably made of materials with high temperature resistance and good thermal conductivity (for example, materials that are resistant to 400°C+ temperature and odorless with good thermal conductivity and suitable strength).

Preferably, the body of the cartridge cup has a taper. Preferably, the diameter of the top opening is greater than that of the bottom end face. The ratio of the diameter difference between the top opening and the bottom end face to the height of the cone, that is, the taper, is K. The diameter of the top opening is expressed as D, the diameter of the bottom end face is expressed as d, and the height of the cone is expressed as H. The taper calculation formula is shown as follows:

$$K=(D-d)/H$$

Where, $0<K\leq0.25$.

Preferably, the cross section of the cartridge cup is round, oval, quadrilateral, polygonal or irregular-shaped. The present invention with the aforementioned structures has the following advantages:

1. The porous structure design of the cover film ensures the passage of airflow and forms a structure easy to be pierced; at the same time, the structure can reliably hold the absorbent cotton and the tobacco particles, thereby making the cartridge of the present invention suitable for varied piercing-type heating smoking set using heating needles, heating sheets, and heating bars as heating elements.

2. Due to the high temperature resistance and heat-transferring side walls of the cartridge cup, the cartridge of the present invention is suitable for the cylinder wall-type heating smoking set.

3. The top opening of the cartridge cup is designed with the flange, thus increasing the hot-sealing area of the cover film, to overcome the problem that the thin cartridge cup wall is difficult to be hot-sealed and the thin cover film is prone to deform. The flange design of the top opening mainly has the following advantages:

(1) A certain fixed area of hot-sealing is ensured, increasing the force bearing point when the cover film is subjectedto heat and pressure, so that the hot-sealing effect is more desirable;

(2) The hot-sealed structure is stable and the cover film is not easy to deform when the cartridge cup is subjected to external force;

(3) When using, the flange part of the cover film is fixed, thus making the center of the cover film easily pierced by force;

(4) In industrial production, with the flange design, the equipment can rapidly sort out the top-bottom end direction of the cartridge, so that the cartridge can be filled in a fast, accurate manner;

(5) When the cartridge is loaded into a cylinder wall-type heating element, the flange structure has a stress surface at the opening, thus ensuring that the lower edge opening of the flange fits more tightly with the end surface of the heating element;

(6) With the flange structure, it is convenient for users to take the cartridge;

(7) The temperature of the cartridge is high after using, so the mechanical structure of the flange can help the corresponding linkage device unload the cartridge.

4. The specific components and content ratios adopted by the HNB tobacco particles of the present invention are designed by taking into account the suction effect, smoke volume, intensity of aroma, naturality of aroma and physiological experience and other effects, thus allowing the HNB tobacco particles to have specific technical effects that are basically identical to that of the traditional cigarette products, in terms of suction effect, smoke volume, intensity of aroma, naturality of aroma and physiological experience.

5. The composite film composed of the sealing film and the cover film is used for packaging. When removing the sealing film during using, the cover film still functions as a packaging film. Among them, PE (polyethylene) layer acts as a heat sealing layer, which is directly connects to the upper plane of the outer flange of the cartridge cup. PE is a thermoplastic material, which can be heat-sealed and fixed after heating at a certain temperature. Thanks to the supporting effect provided by the aluminum layer, the film body is relatively firm with a certain degree of hardness, resistant to light and oxidation, and has a protective effect. The cover film is equipped with gas circulating holes.

6. A non-porous sealing film is used for further sealing and removed when using, wherein the glue layer acts as a bonding layer, which fixes the sealing film at both ends and is easy to be torn off; the sealing layer is used for sealing, with a view to preventing the tobacco particles from leaking out when the cartridge is stored or from being polluted by the external environment, so that the effective components in the tobacco particles can be kept in a long period.

7. The inventor needs to emphasize that in the present invention, a breathable fibrous layer is arranged in the cartridge, and especially anabsorbent cotton is used at the inner bottom of the cartridge, which is an initiative in the cartridge structure of HNB filed. The structure has the following advantages:

(1) The absorbent cotton is fluffy and elastic, so it can rapidly cover the surface of the cover film in a fluffy way after being added to the cartridge cup, thus preventing tobacco particles and powder generated after heating from leaking, while ensuring the smooth passage of air;

(2) A certain amount of absorbent cotton forms a constant elastic supporting space at the bottom of the cartridge cup, so that the density of the HNB particles is relatively stable, which helps deliver even smoke and uniform suction experience;

(3) Absorbent cotton comes from nature and can be degraded easily;

(4) With a high ignition point, absorbent cotton is not easy to be pyrolyzed when heated, thus can avoid the generation of harmful substances or irritating odors;

(5) The cotton filling is an established process, easy to achieve industrial-scale production;

(6) Absorbent cotton has the advantages of low cost, and stable, extensive sources, and is easy to be produced in an industrialized, sustainable way.

8. The inventor needs to emphasize that the cartridge of the application has a taper and has the following advantages when compared with the traditional cylindrical cartridge cup:

(1) good for manufacturing: When materials of the cartridge cup wall are stamped and shaped in the mold via stamping, the taper shape characterized by a small bottom end and a big top end is conducive to stamping and shaping, which is scientific and reasonable with high production efficiency;

(2) good for sorting on assembly line: In the industrial assembly line operation, the cone shape, with the diameter of the bottom end face smaller than that of the top end, helps sort out the top-bottom end direction of the cartridge, which is convenient for subsequent production processes;

(3) good for filling: Since the diameter of the bottom end face is smaller than that of the top end, the filling processes for absorbent cotton and tobacco particles are easier to be realized;

(4) good for film sealing: When the bottom end face is sealed with the film, the base support of the assembly line can better bear the force, thus making the sealing of sealing film easier;

(5) good for loading the cartridge into the smoking set: By pushing the cartridge into the heating cavity of the smoking set, which has a taper corresponding to that of the cartridge, the compatibility between the cartridge and the heating cavity of the smoking set can be significantly improved. In addition, since forces exist between the outer wall of the cartridge cup and the inner wall of the heating cavity, between the lower end of the outer flange at the top opening of the cartridge cup and the upper wall of the heating cavity, and between the upper end of the heating cup cover film and the heating cavity door cover, the compatibility between the cartridge and the heating cavity of the smoking set can be further enhanced, thus raising the air tightness therebetween and delivering better heating and suction effects.

**Description of the Drawings**

For a clearer understanding of the above and other purposes and advantages of the present invention, the preferred embodiment of the present invention is detailed as follows by referring to the drawings,where:

Fig. 1 is an exploded view of the cartridge embodiment of the present invention.

Fig. 2 is a schematic diagram showing the cartridge embodiment of the present invention operated on an assembly line in industrial production.

Fig. 3 is a package schematic diagram of the cartridge embodiment 1 of the present invention.

Fig. 4 is a schematic diagram for the top opening hot sealing of the cartridge embodiment 1 of the present invention.

Fig. 5 is a schematic diagram for the sealing at the bottom end face in the cartridge embodiment 1 of the present invention.

Fig. 6 is a schematic diagram showing the way the cartridge of the present invention is installed in the smoking set heating cavity.

Fig. 7 shows a schematic diagram for the packaging of the cartridge embodiment 2 of the present invention.

Fig. 8 shows a schematic diagram for the cross-sectional shape of the cartridge of the present invention. Symbol of drawings:

| | | |
|---|---|---|
| 1-cartridge | 101-top opening | 102-bottom end face |
| 201-cover film | 2011-aluminum layer | 2012-PE (polyethylene) layer |
| 301-sealing film | 3011-sealing layer | 3012-glue layer |
| 302-sealing film | 3021-sealing layer | 3022-glue layer |
| 4-absorbent cotton | 5-tobacco particles | |

Detailed description of the embodiment

Embodiment 1:

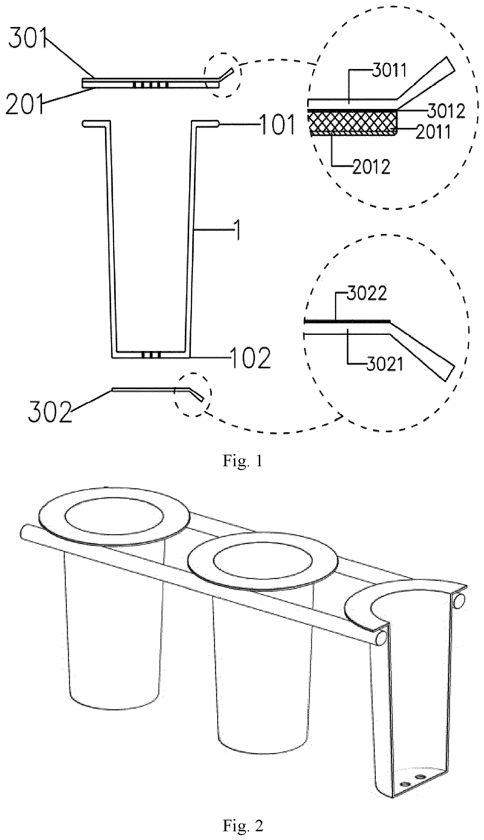

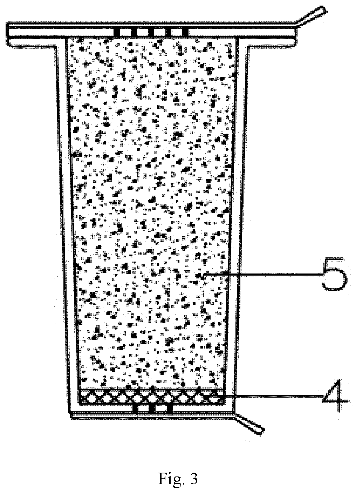

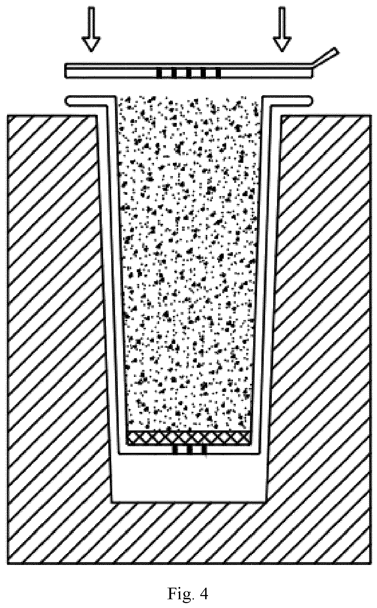

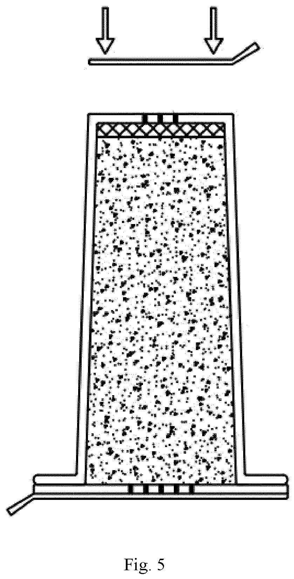

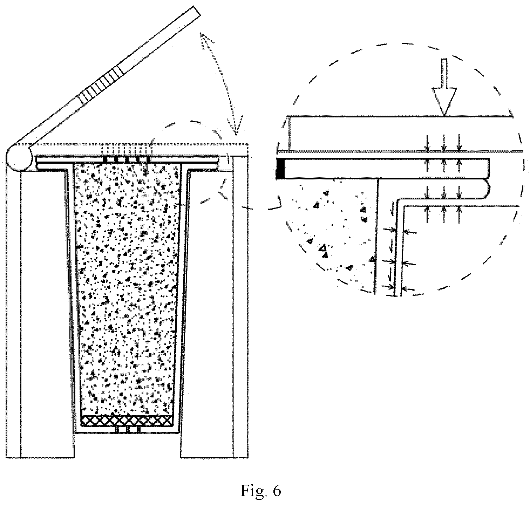

As shown in Figs. 1-6, the cartridge presented in the embodiment 1 of the present invention is composed of a cartridge packaging housing and HNB tobacco particles 5 contained therein. The cartridge packaging housing is composed of a cartridge cup 1, a cover film 201, a sealing film 301 and a sealing film 302; The cartridge cup is provided with a top opening 101 and a bottom end face 102; and the bottom end face is provided with gas circulating holes; the top opening 101 is sealed by the cover film 201 and the sealing film 301; the cover film 201 is provided with multiple gas circulating holes; the bottom end face 102 is sealed by the sealing film 302. The cartridge cup 1 can be made from aluminum stamped under high pressure. The aluminum layer 2011 of the cover film 201 can be made of aluminum foil materials. The sealing layer 3011 of the sealing film 301 and the sealing layer 3021 of the sealing film 302 can be made of thin plastic materials.

As shown in Figs. 1-6, the top opening 101 of the cartridge cup 1 is provided with an outer flange radially protruding outward along the cartridge cup 1. Such a structure is conducive to enlarge the heat-sealing fixing area of the cover film 201 at the top opening 101, so that the cover film 201 can be heat-sealed at the top opening 101 of the cartridge cup 1 more firmly.

The cartridge of embodiment 1 with the above structure has a cartridge packaging housing, which can effectively ensure the quality of the tobacco particles contained therein, and make the cartridge easy to use with guaranteed suction effect.

The cartridge of the embodiment 1 is produced by the follow steps: first, the cartridge cup 1 is placed upright with the top opening 101 facing upwards, and then the cup bottom is filled with the absorbent cotton 4 from the top opening by air stamping, so that the absorbent cotton fully covers the bottom under the impact of air pressure and its own fluffy volume; and then the cartridge cup 1 is filled with tobacco particles 5 from the top opening to downward; and then the composite film that integrates the cover film 201 and the sealing film 301 is hot-sealed on the out flange of the top opening; at last, the sealing film 302 is sealed at the bottom end face 102, to complete the preparation of the cartridge.

As for the non-piercing type cylinder wall heating smoking set, when using the cartridge, sealing films at the both ends of the cartridge are removed first, and then the cartridge is loaded into the non-piercing type cylinder wall heating smoking set, whose inner diameter of cavity wall matches the outer diameter of the cartridge, thus the cartridge can be used conveniently; As for the piercing-type heating smoking set, when using the cartridge, sealing films at the both ends of the cartridge are removed first, and then the cartridge is loaded into the heating cavity of the smoking set with a piercing-type heating element (such as heating needle), enabling the heating element to pierce the cover film from the top end and enter into the pierced cartridge cup 1 of the cartridge, thus the tobacco particles 5 can be heated.

As for the cartridge provided in the embodiment 1, the HNB tobacco particles 5 used comprises microcrystalline celluloses, tobacco powder, low-temperature pyrolysis products of tobacco, a physiological taste enhancer of tobacco, an atomizing agent, a tobacco flavor, and a flavor solvent. Wherein, by weight fraction, the HNB tobacco particles comprise 8-20 parts of the microcrystalline celluloses, 50-80 parts of the tobacco powder, 0.5-2 parts of the low-temperature pyrolysis products of tobacco, 1 part of the physiological taste enhancer of tobacco, 8-30 parts of the atomizing agent, 0.1-2 parts of the tobacco flavor and 1-10 parts of the flavor solvent. The low-temperature pyrolysis products of tobacco is a product of tobacco pyrolysis at 220-280°C by far-infrared heating. The physiological taste enhancer of tobacco is composed of alkaloid and water. The alkaloid is nicotine or nicotine salt, and if nicotine is selected, the physiological taste enhancer of tobacco is composed of 20-40% of the nicotine and 60-80% of the water; if nicotine salt is selected, the physiological taste enhancer of tobacco is composed of 5-20% nicotine salt and 80-95% of the water. The atomizing agent can be selected from polyol or an ester derivative of polyol. The atomizing agent can be selected from vegetable glycerin, propylene glycol or glyceryl triacetate. The flavor solvent can be selected from vegetable glycerin, propylene glycol or glyceryl triacetate.

The specific components and content ratios used by the HNB tobacco particles 5 of the present invention allow the HNB tobacco particles to have specific technical effects that are basically identical to that of the traditional cigarette products, in terms of suction effect, smoke volume, intensity of aroma, naturality of aroma and physiological experience. In particular, when combined with the cartridge packaging housing with the above specific structures, the moisture and aroma substances in the tobacco particles can be better retained, thereby long ensuring the quality of the cartridge and remarkably improving the suction effect of the cartridge.

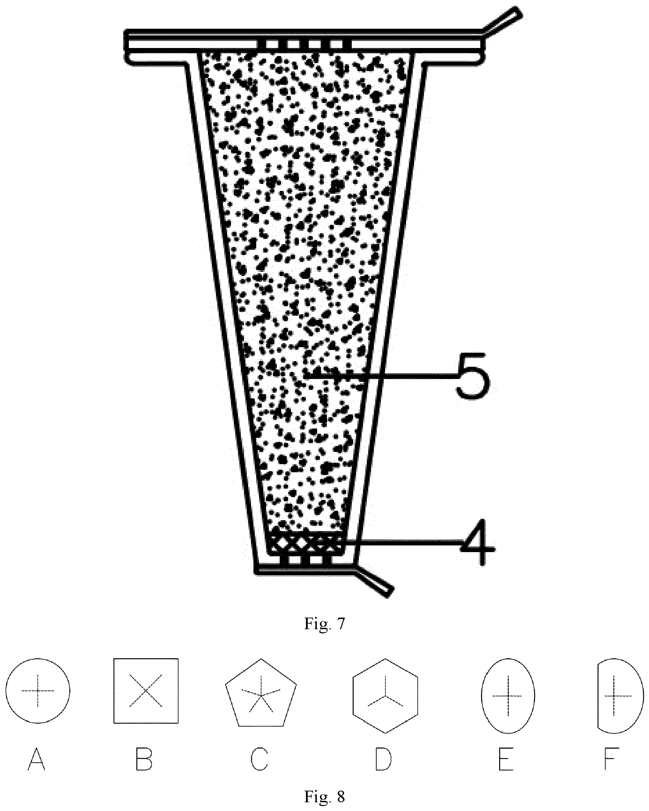

As shown in example A of Fig. 8, the body of the cartridge cup 1 is cone-shaped, with circular cross section. Its taper is preferably 0.025.

Embodiment 2:

As shown in Fig.7, the cartridge provided in the embodiment 1 of the present invention is composed of a cartridge packaging housing and HNB tobacco particles 5 contained therein. The cartridge cup is made from ceramics cast under high temperature. The aluminum layer in the cover film uses aluminum coated paper and the sealing layer in the sealing film uses thin paper.

The cross section of the body of the cartridge cup can be varied shapes as shown in Fig. 8.

Moreover, it should be further emphasized that thanks to the careful study made by the inventor, the present invention presents a cup body that has a taper, which can deliver a better compatibility between the cartridge and the heating cavity of the smoking set as well as a higher air tightness therebetween when compared with the traditional cylindrical cup body, thus remarkably improving the heating and suction effects of the cartridge. Based on this, the inventor found through further tests and studies that when the body of the cartridge cup is of certain taper, the compatibility between the cartridge and the heating cavity of the smoking set as well as the gas tightness therebetween can be further improved, thus delivering a better using performance. The inventor needs to further emphasize that as shown in Fig. 8, the application provides the cartridge with a variety of cross-section shapes. In practical use, the cross section shape of the cartridge, including oval, quadrilateral, polygonal or irregular shape, can be selected according to the actual situation. A proper shape ensures the compatibility between the cartridge and the heating cavity of the smoking set, while helps to improve the design freedom of the appearance and internal structure of the smoking set.

The above-mentioned are only the preferred embodiments of the present invention. It should be stressed that various changes and modifications made to the present invention by those of ordinary skill in the art without departing from the spirit of the present invention will fall into the protection scope of the present invention.

**Claims**

1. An HNB cartridge structure, composed of a cartridge packaging housing and HNB tobacco particles (5) contained therein, **characterized in that** the cartridge packaging housing is composed of a cartridge cup (1), a cover film (201) and sealing films (301, 302), the cartridge cup (1) is provided with a top opening (101) and a bottom end face (102), the cover film (201) and the bottom end face (102) are provided with gas circulating holes, the top opening (101) is sealed by the cover film (201), the outer surface of the cover film (201) and the bottom end face (102) are sealed with the sealing films (301, 302).

2. The cartridge structure according to claim 1, **characterized in that** the HNB tobacco particles comprise microcrystalline celluloses, a tobacco powder, low-temperature pyrolysis products of tobacco, a physiological taste enhancer of tobacco, an atomizing agent, a tobacco flavor and a flavor solvent.

3. The cartridge structure according to claim 2, **characterized in that** by weight fraction, the HNB tobacco particles comprise 8-20 parts of the microcrystalline celluloses, 50-80 parts of the tobacco powder, 0.5-2 parts of the low-temperature pyrolysis products of tobacco, 1 part of the physiological taste enhancer of tobacco, 8-30 parts of the atomizing agent, 0.1-2 parts of the tobacco flavor and 1-10 parts of the flavor solvent, the low-temperature pyrolysis products of tobacco are generated by tobacco pyrolysis with far-infrared heating under 220-280 °C.

4. The cartridge structure according to claim 2 or 3, **characterized in that** the physiological taste enhancer of tobacco is composed of water and alkaloid, the alkaloid is selected from nicotine or nicotine salt, wherein

if the nicotine is selected, the physiological taste enhancer of tobacco is composed of 20-40% of the nicotine and 60-80% of the water;
if the nicotine salt is selected, the physiological taste enhancer of tobacco is composed of 5-20% of the nicotine salt and 80-95% of the water.

5. The cartridge structure according to claim 2 or 3, **characterized in that** the atomizing agent is selected from polyols or ester derivatives thereof, and the flavor solvent is selected from vegetable glycerin, propylene glycol or glyceryl triacetate.

6. The cartridge structure according to claim 5, **characterized in that** a atomizing agent is selected from vegetable glycerin, propylene glycol or glyceryl triacetate.

7. The cartridge structure according to claim 1, **characterized in that** the top opening of the cartridge cup is provided with an outer flange radially protruding outward along the cartridge cup.

8. The cartridge structure according to claim 1 or 7, **characterized in that** the top opening (101) of the cartridge cup (1) is heat-sealed by the cover film (201).

9. The cartridge structure according to claim 1 or 7, **characterized in that** a breathable fibrous layer is arranged on the bottom end face (102) in the cartridge cup (1).

10. The cartridge structure according to claim 9, **characterized in that** the breathable fibrous layer is an absorbent cotton (4).

11. The cartridge structure according to claim 8, **characterized in that** the cover film (201) and the sealing film (301) at the top opening (101) form an integrated composite film that is heat-sealed at the top opening (101) of the cartridge cup.

12. The cartridge structure according to claim 11, **characterized in that** the cover film (201) is a thermoplastic multi-layer composite made of a PE layer and an aluminum layer, wherein the PE layer acts as a heat sealing layer.

13. The cartridge structure according to claim 12, **characterized in that** the aluminum layer of the thermoplastic multi-layer composite is aluminum coated paper or aluminum foil.

14. The cartridge structure according to claim 11, **characterized in that** the sealing films (301, 302) are adhesive multi-layer composite composed of a glue layer and a sealing layer, wherein the glue layer acts as a bonding layer.

15. The cartridge according to claim 14, **characterized in that** the sealing layer of the adhesive multi-layer composite is thin paper or thin plastic.

16. The cartridge structure according to claim 1, **characterized in that** the body of the cartridge cup (1) has a taper.

17. The cartridge structure according to claim 16, **characterized in that** the top opening (101) is larger than the bottom end face (102) by area.

18. The cartridge structure according to claim 16 or 17, **characterized in that** the body of the cartridge cup (1) is frustum cone-like, wherein the ratio of the diameter difference between the top opening and the bottom end face to the height of a cone, that is, the taper value of the body of the cartridge cup (1) is K, where $0 < K \leq 0.25$.

19. The cartridge structure according to claim 1 or 16, **characterized in that** the cross section of the cartridge cup (1) is round, oval, quadrilateral, polygonal or irregular-shaped.

Fig. 1

Fig. 2

Fig. 3

Fig. 4

Fig. 5

Fig. 6

Fig. 7

A     B     C     D     E     F

Fig. 8

## INTERNATIONAL SEARCH REPORT

International application No.

**PCT/CN2019/085099**

### A. CLASSIFICATION OF SUBJECT MATTER

A24F 47/00(2006.01)i; A24B 3/14(2006.01)i

According to International Patent Classification (IPC) or to both national classification and IPC

### B. FIELDS SEARCHED

Minimum documentation searched (classification system followed by classification symbols)

A24F47,A24B3

Documentation searched other than minimum documentation to the extent that such documents are included in the fields searched

Electronic data base consulted during the international search (name of data base and, where practicable, search terms used)

VEN, SIPOABS, CNABS, CNTXT, CNKI: 广东精彩国际生物科技有限公司, 山东精彩香料科技开发有限公司, 钱剑冰, SHANDONG WONDERFUL PERFUME TECHNOLOGY, SHANDONG JINGCAI SPICE TECH DEVELOPMENT CO LTD, capsule, shell, seal+ film, air, flow+, cup, 烟弹, 烟草胶囊, 气溶胶生成制品, 密封, 膜, 密封薄膜, 气孔, 气流, 气体, 流通, 流动, 壳体, 外壳, 杯, 烟?杯

### C. DOCUMENTS CONSIDERED TO BE RELEVANT

| Category* | Citation of document, with indication, where appropriate, of the relevant passages | Relevant to claim No. |
|---|---|---|
| PX | CN 109275963 A (SHANDONG WONDERFUL SPICE TECHNOLOGY DEVELOPMENT CO., LTD.) 29 January 2019 (2019-01-29)<br>entire document | 1-19 |
| PX | CN 109363243 A (LENG, Chaoyang) 22 February 2019 (2019-02-22)<br>paragraphs 22-27 | 1 |
| Y | CN 108936816 A (SHANDONG WONDERFUL SPICE TECHNOLOGY DEVELOPMENT CO., LTD.) 07 December 2018 (2018-12-07)<br>paragraphs 4 and 14-30, and figures 1-3 | 1-19 |
| Y | CN 108567172 A (HESTIA SHENZHEN BIOTECHNOLOGY CO., LTD.) 25 September 2018 (2018-09-25)<br>see paragraphs 20, 24 and 28-30 | 1-19 |
| Y | CN 108077992 A (SHANDONG WONDERFUL SPICE TECHNOLOGY DEVELOPMENT CO., LTD.) 29 May 2018 (2018-05-29)<br>see paragraphs 20-25, and claims 1-10 | 2-6 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 September 2019** | **27 September 2019** |

| Name and mailing address of the ISA/CN | Authorized officer |
|---|---|
| **China National Intellectual Property Administration**<br>**No. 6, Xitucheng Road, Jimenqiao Haidian District, Beijing**<br>**100088**<br>**China** | |
| Facsimile No. **(86-10)62019451** | Telephone No. |

Form PCT/ISA/210 (second sheet) (January 2015)

**INTERNATIONAL SEARCH REPORT**

| | International application No. |
|---|---|
| | **PCT/CN2019/085099** |

**C.    DOCUMENTS CONSIDERED TO BE RELEVANT**

| Category* | Citation of document, with indication, where appropriate, of the relevant passages | Relevant to claim No. |
|---|---|---|
| Y | CN 2503762 Y (ZHANG, Hongjun) 07 August 2002 (2002-08-07)<br>see embodiments 1 and 2, and figure 2 | 7-15 |
| Y | CN 108652077 A (CHINA TOBACCO YUNNAN INDUSTRIAL CO., LTD.) 16 October 2018 (2018-10-16)<br>see paragraph 37 and embodiments 3 and 4, and figure 1 | 11-15, 16-17 |

Form PCT/ISA/210 (second sheet) (January 2015)

**INTERNATIONAL SEARCH REPORT**
Information on patent family members

International application No.

**PCT/CN2019/085099**

| Patent document cited in search report | | | Publication date (day/month/year) | Patent family member(s) | | | Publication date (day/month/year) |
|---|---|---|---|---|---|---|---|
| CN | 109275963 | A | 29 January 2019 | None | | | |
| CN | 109363243 | A | 22 February 2019 | None | | | |
| CN | 108936816 | A | 07 December 2018 | None | | | |
| CN | 108567172 | A | 25 September 2018 | WO | 2019042144 | A1 | 07 March 2019 |
| CN | 108077992 | A | 29 May 2018 | WO | 2019119874 | A1 | 27 June 2019 |
| | | | | CN | 108077992 | B | 18 January 2019 |
| CN | 2503762 | Y | 07 August 2002 | None | | | |
| CN | 108652077 | A | 16 October 2018 | None | | | |

Form PCT/ISA/210 (patent family annex) (January 2015)